# EUROPEAN PATENT SPECIFICATION

(11) **EP 0 868 908 B1**
(45) Date of publication and mention of the grant of the patent: **16.06.2004**
(21) Application number: 98302071.0
(22) Date of filing: 19.03.1998
(51) Int. Cl.: A61K 7/48

(54) **Cosmetic material**
Kosmetisches Mittel
Composition cosmétique

(30) Priority: 19.03.1997 JP 8463697
(43) Date of publication of application: 07.10.1998
(73) Proprietor: Kabushiki Kaisha Yakult Honsha, Minato-ku, Tokyo 105-8660 (JP)
(72) Inventor: Yoshikawa, Satoshi c/o Kabushiki Kaisha Yakult Hon, Tokyo 105-8660 Japan (JP); Tagami, Gonichi c/o Kabushiki Kaisha Yakult Honsha, Tokyo 105-8660 Japan (JP); Yamamoto,Shozi c/o Kabushiki Kaisha Yakult Honsha, Tokyo 105-8660 Japan (JP); Hiraki, Yoshio c/o Kabushiki Kaisha Yakult Honsha, Tokyo 105-8660 Japan (JP)
(74) Representative: Ablewhite, Alan James

(56) References cited:
- EP-A- 0 676 194
- EP-A- 0 826 366
- WO-A-94/27569
- WO-A-96/25917
- WO-A-96/37179
- FR-A- 2 735 688
- US-A- 5 624 664
- PATENT ABSTRACTS OF JAPAN vol. 12 (C & JP 08 217622 A (YAKULT HONSHA CO)

## Description

### BACKGROUND OF THE INVENTION

### Field of the Invention

The present invention relates to a cosmetic material having an excellent moisture-retaining effect and skin turnover acceleration function, and having a good feeling after use.

### Discussion of the Background

Polyfunctional alcohols such as glycerine and propylene glycol, sugars such as sorbitol and multitol, amino acids, and polymer substances such as hyaluronic acid and chondroitin sulfate have been known in the art as moisture-retaining substances to be blended in cosmetic materials for skin. The supernatant of a culture medium of lactic acid bacteria is also known to have a moisture-retaining function.

On the other hand, it is also known that α -hydroxy acid (AHA) not only has a moisture-retaining effect but also a skin turnover acceleration function and curing effect for ichthyosis. Accordingly, the material has come to be regarded as a material of cosmetics (Japanese Patent Laid-Open No. 5-139947).

Nevertheless, when used alone, the moisture-retaining function of the supernatant of lactic acid bacteria or α-hydroxy acid is not always satisfactory, and yet a sufficient amount of moisture retaining function can not be obtained when the blending quantity is small. Also, blending them in a large amount results in a clammy feeling after use and increases the cost.

Alpha-hydroxyacids are used also in FR-A-2 735 688, WO-A-96/25917, WO-A-94/27569, EP-A-0 676 194 and WO-A-96/37179. Of the alpha-hydroxyacids, the most simple alpha-hydroxyacids, which are glycolic acid and lactic acid, are most preferably used in these documents. A variety of weight percentages and ratios of alpha-hydroxyacids are used in these documents for such purposes as whitening skin, or for providing good 'and beautiful skin. These documents, however, do not disclose the use of a culture supernatant of lactic acid bacteria, and do not teach combining lactic acid, glycolic acid and a culture supernatant of lactic acid bacteria for improved moisture retention.

### [Problems to be Solved by the Invention)

Accordingly, the object of the present invention is to provide a cosmetic material having an excellent moisture retaining function and skin turnover acceleration function, which is inexpensive and has a good feeling after use.

### SUMMARY OF THE INVENTION

We have found from our collective studies that, contrary to our expectations, a cosmetic material with an excellent moisture retaining effect as well as a good feeling after use can be obtained from the synergetic effect of blending a lactic acid and a glycolic acid, which are all α-hydroxy acids, in a specific proportion. Moreover, when a supernatant of a culture medium of lactic acid bacteria was combined with the mixture described above, it was found that a cosmetic material with a much better feeling after use and an improved moisture retaining effect could be obtained, thereby completing the present invention.

Accordingly, a first aspect of the present invention provides a cosmetic material containing (A) lactic acid, (B) glycolic acid and (C) a supernatant of a culture medium of lactic acid bacteria, wherein the weight ratio of (A) : (B) is from 2 : : 8 to 9 : 1.

The present invention also provides a method for preparing the above mentioned cosmetic material.

### BRIEF DESCRIPTION OF THE DRAWINGS

Fig. 1 is a graph showing the relationship between the mixing ratio of lactic acid and glycolic acid and the moisture retaining properties with skin.
Fig. 2 is a graph showing the relationship between the amount of lactic acid and the supernatant of the culture medium of lactic acid bacteria blended and the moisture retaining properties with skin.

### DETAILED DESCRIPTION OF THE INVENTION

(A) lactic acid and (B) glycolic acid produced by any method, including either a synthetic method or a fermentation method, can be used as the materials of the present invention. Any form of D-, L- or DL-lactic acid can also be used.

Lactic acid and glycolic acid for use in the present invention may either be in their native forms or processed by various methods such as heat treatment.

In the present invention, it is preferable that heat treatment is performed after the pH values of the lactic acid and glycolic acid are adjusted higher than their desired values by adding a base so that the lactic acid and glycolic acid are stabilized at nearly neutral conditions and have an improved soft feeling to the skin (Japanese Patent Laid-Open No. 8-217622).

It is preferable to adjust the pH value to 8 to 9 while heat treatment is preferably applied for 1 hour or more at 80 to 90°C. This treatment allows the lactic acid and the glycolic. acid to be blended-with a moisture retaining agent such as hyaluronic acid or a fermentation broth of lactic acid bacteria that is effective at around neutral conditions. The cosmetic materials prepared as described above can be applied to many uses such as milky lotions, moisture retaining creams, cleansing creams, massage creams, face cleaning creams, lotions, packs, beauty lotions, and the like.

In the present invention, the blending ratio of (A) lactic acid to (B) glycolic acid is important. A weight ratio of 2 : 8 to 9 : 1, more preferably 3 : 7 to 8 : 2 and most preferably 4 : 6 to 7 : 3 is recommended to obtain a synergetic moisture retaining effect.

The moisture retaining effect is further enhanced by adding the supernatant of the culture medium of lactic acid bacteria to the mixture of lactic acid and glycolic acid described above. Although the supernatant of the culture medium of lactic acid bacteria used in the present invention refers to "whey (2)" described in "Standards for Nonstandardized Ingredients of Cosmetic Materials", they are not limited thereto but can be obtained by various methods including the methods as described in Japanese Patent Publication No. 2-24247. In other words, any products obtained by fractionating milk serum from the fermentation product, which is produced by lactic acid fermentation of a culture medium mainly containing animal milk by seeding the culture medium lactic acid bacteria, can be used.

A native supernatant of the culture medium of lactic acid bacteria may be used or the supernatant may be used after eliminating its aroma component by heating it under a reduced pressure.

Examples of lactic acid bacteria to be used in the lactic acid fermentation are those commonly used in the preparation of lactic acid bacteria beverages and fermented milk such as, Lactobacillus acidophilus, Lactobacillus bulgaricus, Lactobacillus casei, Streptococcus thermophilus and Lactococcus lactis. The animal milk to be used as a culture medium may be human milk, cow milk or goat milk, and the like, or reduced milks from skim milk or powdered milk, and the like may also be used.

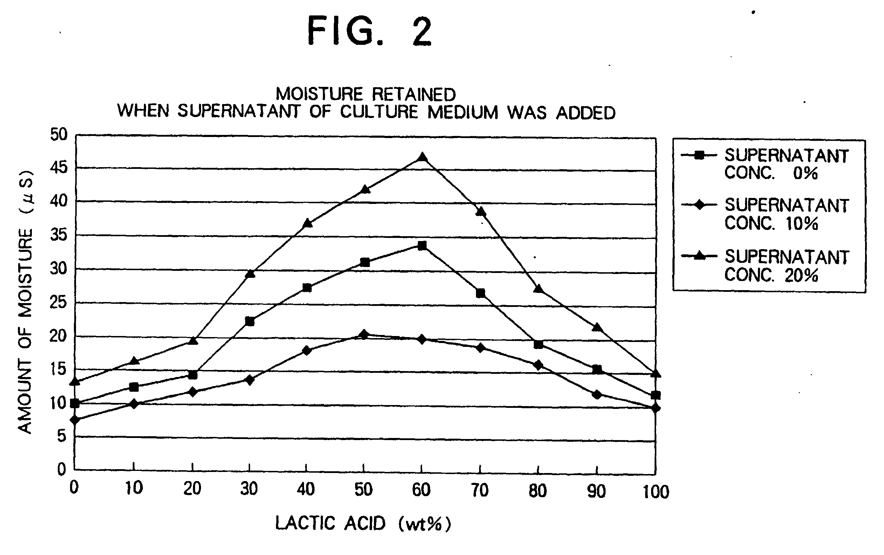

The present inventors researched the preferable ratio between the mixture of lactic acid and glycolic acid and the supernatant of the culture medium of lactic acid bacteria, but it was found that this ratio has little relation to the moisture retaining effect; instead, the higher the concentration of the supernatant of the culture medium of lactic acid bacteria in the cosmetic material composition, the more the moisture retaining effect is enhanced (Fig. 2).

It is therefore preferable that the supernatant of the culture medium of lactic acid bacteria is blended with the cosmetic material composition in a ratio of 1 to 80 % by weight, more preferably 3 to 40 % by weight and most preferably 5 to 20 % by weight. The preferable ratio between the.lactic acid and the glycolic acid remains the same as the range described above even when the supernatant of the culture medium of lactic acid bacteria is added to the composition.

The preferable pH range of the cosmetic material according to the present invention is, taking the softening effect with skin and stability of the product into account, 3.5 to 8.0, especially 5.0 to 7.0.

The cosmetic material according to the present invention can be produced by conventional methods. Various modifications of the material are possible by adding ingredients conventionally used in cosmetic material to produce milky lotions, moisture retaining creams, cleansing creams, massage creams, face cleaning creams, lotions, packs and beauty lotions, and the like.

### EXAMPLES

Methods of the present invention are illustrated with reference to the following examples, but the invention is not intended to be limited only thereto.

### Experiment 1

The effect of the mixing ratio of lactic acid and glycolic acid on the moisture retaining property of the skin was investigated.

### (Method)

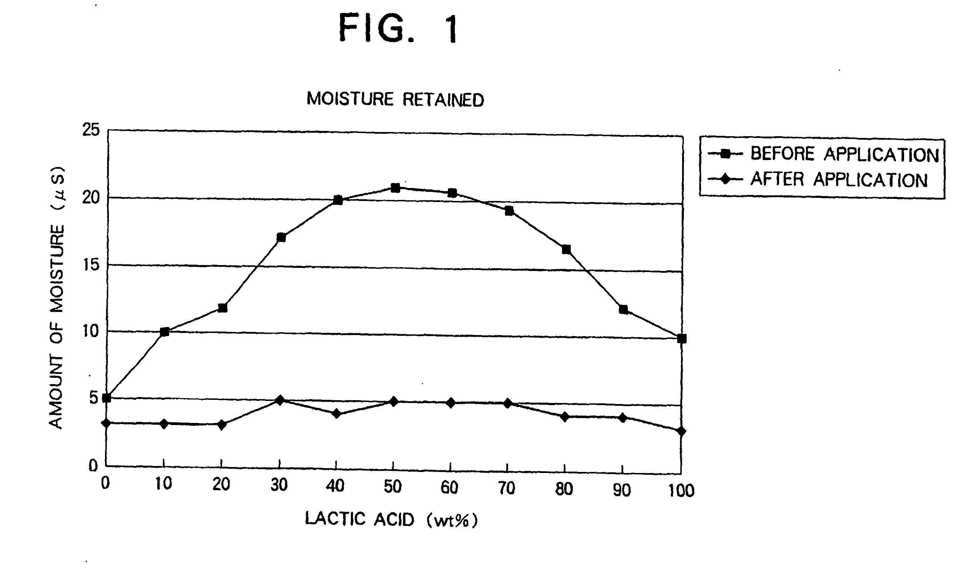

Eleven samples of aqueous solutions containing 5% of α - hydroxy acids with different mixing ratios of lactic acid and glycol acid were prepared (Fig. 1) and the pH of each solution was adjusted to 6.0 with sodium hydroxide. These samples prepared were applied on the volar side of the forearms of people (5 people) with dry skin. After 30 minutes, the moisture content of the skin at the portion with the application was measured with a moisture meter (Skicon type 200, made by IBS Co.). The moisture content of a person without the application was also measured. The same experiment was repeated three times to obtain a mean value.

### (Results)

The synergetic effect of lactic acid with glycolic acid was observed within the range of 10 to 90 % by weight of lactic acid in the α -hydroxy acid mixture (Fig. 1).

### Experiment 2

The effects of lactic acid, glycolic acid and the supernatant of the culture medium of lactic acid bacteria on the moisture retaining properties of the skin were investigated.

### (Method)

The supernatant of the culture medium of lactic acid bacteria was added in a proportion of 0, 10 and 20 % by weight to a sample prepared as in Experiment 1 and the pH of each solution was adjusted to 6.0 with sodium hydroxide (Fig. 2). These samples prepared were applied on the volar side of the forearms of people with dry skin (5 people) and, after 30 minutes, the moisture content of the skin at the applied portion was measured with a moisture meter (Skicon type 200, made by IBS Co.). The moisture content of the skin where aqueous solutions containing 0, 10 and 20 % by weight of the supernatant of the culture medium of lactic acid bacteria without a -hydroxy acids (control sample) was applied, and the moisture content of skin without any application, were also measured.

### (Results)

Among the aqueous solutions of lactic acid and glycolic acid, a stronger synergetic effect was obtained with a proportion of lactic acid of 30 to 80 % by weight. The effect was especially remarkable with a proportion of 40 to 60 % by weight (Fig. 2) .

### Example 1: Preparation of milky lotion blended with lactic acid and glycolic acid.

The milky lotion was prepared according to the following prescription:

**Table 1**

| (Ingredient) | | (% by weight) |
|---|---|---|
| (1) | Liquid paraffin | 5 |
| (2) | Octyldodecyl myristate | 5 |
| (3) | Squalene | 3 |
| (4) | Cetanol | 1.0 |
| (5) | Stearic acid | 1.0 |
| (6) | Butyl paraben | 0.1 |
| (7) | POE (20) sorbitan monostearate | 2 |
| (8) | Glyceryl monostearate | 2 |
| (9) | 1,3-butylenegycol | 5 |
| (10) | Methyl paraben | 0.2 |
| (11) | Supernatant of culture medium of lactic acid bacteria | 10 |
| (12) | Glycolic acid | 0.2 |
| (13) | Lactic acid | 0.3 |
| (14) | Perfume | 0.1 |
| (15) | Sodium hydroxide | appropriate quantity |
| (16) | Purified water | amount to adjust total weight ratio to 100 |

### (Method of preparation)

A solution prepared by dissolving (9) and (10) in (16) and a solution prepared by adjusting the pH of the mixed solution of (11) to (13) to 6.5 with sodium hydroxide (15) were mixed with a solution prepared by mixing and dissolving (1) to (8) at 80°C . After cooling the solution obtained to 50°C, (14) was added to the solution with mixing.

### Example 2: A lotion was produced according to the following prescription:

**Table 2**

| (Ingredient) | | (% by weight) |
|---|---|---|
| (1) | Ethyl alcohol | 5 |
| (2) | EDTA2Na | 0.1 |
| (3) | Glycerine | 5 |
| (4) | 1,3-Butyleneglycol | 2.5 |
| (5) | Methylparaben | 0.1 |
| (6) | Supernatant of culture medium of lactic acid bacteria | 10 |
| (7) | Glycolic acid | 0.25 |
| (8) | Lactic acid | 0.25 |
| (9) | Sodium hydroxide | appropriate quantity |
| (10) | POE (40) hydrogenated castor oil | 0.25 |
| (11) | Perfume | 0.05 |

### (Method of preparation)

A solution prepared by dissolving (1) to (6) in an appropriate amount of purified water, a mixture of (7) to (9) prepared by adjusting the pH to 6, and a solution prepared by dissolving (10) and (11) in an appropriate amount of water were mixed with each other followed by adjusting the total weight ratio to 100 by adding purified water.

### Example 3: Preparation of a cream blended with lactic acid and glycolic acid.

A cream was prepared according to the following prescription:

**Table 3**

| (Ingredient) | | (% by weight) |
|---|---|---|
| (1) | Liquid paraffin | 6 |
| (2) | Squalene | 15 |
| (3) | Vaseline | 9 |
| (4) | Bees wax | 4 |
| (5) | Stearic acid | 0.5 |
| (6) | Cetanol | 2 |
| (7) | POE (20) sorbitan monostearate | 3 |
| (8) | Glycerine monostearate | 2 |
| (9) | Butyl paraben | 0.1 |
| (10) | 1,3-Butyleneglycol | 3 |
| (11) | Xanthan gum | 0.1 |
| (12) | Methyl paraben | 0.2 |
| (13) | Supernatant of culture medium of lactic acid bacteria | 5 |
| (14) | Glycolic acid | 0.3 |
| (15) | Lactic acid | 0.2 |
| (16) | Perfume | 0.2 |
| (17) | Sodium hydroxide | appropriate quantity |
| (18) | Purified water | amount to adjust total weight ratio to 100 |

### (Method of preparation)

A solution was prepared by mixing (1) to (9) with melting at 80°C as Phase A. Then, a solution prepared by dissolving (10) to (12) in a part of (18) and a solution prepared by dissolving (13) to (15) in the remainder of (18) and then adjusting the solution to a pH of 6.0 with (17) were mixed and the mixture was heated to 80°C to prepare Phase B. After mixing A with B, the solution is cooled to 50°C followed by the addition and mixing of (16).

### Example 4: Preparation of a pack blended with lactic acid and glycolic acid.

A pack was prepared according to the following prescription:

**Table 4**

| (Ingredient) | | (% by weight) |
|---|---|---|
| (1) | Ethanol | 10 |
| (2) | Polyvinyl alcohol | 15 |
| (3) | POE (5) POP glycol (35) | 1 |
| (4) | 1,3-butyleneglycol | 6 |
| (5) | Methyl paraben | 0.2 |
| (6) | Supernatant of culture medium of lactic acid bacteria | 20 |
| (7) | Glycolic acid | 3 |
| (8) | Lactic acid | 2 |
| (9) | Perfume | 0.2 |
| (10) | Sodium hydroxide | appropriate quantity |
| (11) | Purified water | amount to adjust total weight ratio to 100 |

### (Method of preparation)

A solution prepared by dissolving (2) to (5) in a part of (11) and a solution prepared by dissolving (6) to (8) in the remainder of (11) and adjusting the solution to a pH of 5.5 with sodium hydroxide (10) were mixed followed by heating the mixture to 70°C. After cooling to 40°C, (1) and (9) were' added to the. solution with mixing.

### Example 5: Preparation of a beauty lotion blended with lactic acid and glycolic acid.

A beauty lotion was prepared according to the following prescription:

**Table 5**

| (Ingredient) | | (% by weight) |
|---|---|---|
| (1) | Ethanol | 8 |
| (2) | POE (50) hydrogenated castor oil | 0.5 |
| (3) | Carboxyvinyl polymer | 0.5 |
| (4) | Glycerine | 10 |
| (5) | 1,3-Butyleneglycol | 10 |
| (6) | Methylparaben | 0.2 |
| (7) | Supernatant of culture medium of lactic acid bacteria | 40 |
| (8) | Glycolic acid | 2 |
| (9) | Lactic acid | 3 |
| (10) | Perfume | 0.2 |
| (11) | Sodium hydroxide | appropriate quantity |
| (12) | Purified water | amount to adjust total weight ratio to 100 |

### (Method of preparation)

A solution prepared by mixing (7) to (9) and the adjusting the solution to a pH of 5.0 with sodium hydroxide (11) was mixed with a solution prepared by mixing (1) to (6) followed by dissolving at 40°C, then (10) was added with mixing.

### Experiment 3

Beauty lotions were prepared according to the four prescriptions shown in Table 6 to study the effect of the lactic acid/glycolic acid ratio on moisture retaining properties. The samples were applied on the volar side of the forearms of people with dry skin (5 people) in a quantity of 60 µ 1/16cm² and the moisture content of each portion was measured as described in Experiment 1.

**Table 6**

| Ingredients | Comparative materials | | | Present Invention |
|---|---|---|---|---|
| | 1 | 2 | 3 | 1 |
| Ethyl alcohol | 5.00 | 5.00 | 5.00 | 5.00 |
| EDTA·2Na | 0.10 | 0.10 | 0.10 | 0.10 |
| Glycerin | 5.00 | 5.00 | 5.00 | 5.00 |
| 1,3-butyleneglycol | 2.50 | 2.50 | 2.50 | 2.50 |
| Methylparaben | 0.1 | 0.1 | 0.1 | 0.1 |
| Supernatant of culture medium of lactic acid bacteria | 10.00 | 10.00 | 10.00 | 10.00 |
| Glycolic acid | 0 | 0.5 | 0 | 0.25 |
| Lactic acid | 0 | 0 | 0.5 | 0.25 |
| Sodium hydroxide | a.q* | a.q | a.q | a.q |
| POE(50) hydrogenated castor oil | 0.25 | 0.25 | 0.25 | 0.25 |
| Perfume | 0.05 | 0.05 | 0.05 | 0.05 |
| Purified water | All adjusted to pH 6 | | | |
| Water content (µ S) | 23 | 26 | 30 | 47 |

| | | | | |
|---|---|---|---|---|
| * a.q : appropriate quantity | | | | |

As hitherto described, good moisture retaining properties were obtained when both lactic acid and glycolic acid were added as compared with the cases where any of the a -hydroxy acids were added or either lactic acid or glycolic acid was added alone.

According to the present invention, a composition having high moisture retaining properties can be obtained by adjusting the ratio between lactic acid and glycolic acid even when they are added in a small amount. Higher moisture retaining properties can be also obtained with smaller amounts of α - hydroxy acids when the supernatant of the culture medium of lactic acid bacteria is added to the composition.

This invention enables the production of cosmetic materials having good moisture retaining properties and feeling after use at a low production cost, and enhances the turnover effect of skin.

## Claims

1. A cosmetic material containing (A) lactic acid, (B) glycolic acid and (C) a supernatant of a culture medium of lactic acid bacteria, wherein the weight ratio of (A) : (B) is from 2 : 8 to 9 : 1.

2. A cosmetic material according to Claim 1, wherein the weight ratio of (A) : (B) is from 3 : 7 to 8 : 2.

3. A cosmetic material according to Claim 1, wherein the weight ratio of (A): (B) is from 4 :6 to 7:3.

4. A cosmetic material according to Claim 1, wherein (C) is present in an amount of 3 to 40 % by weight.

5. A cosmetic material according to Claim 1, wherein (C) is present in an amount of 5 to 20 % by weight.

6. A cosmetic material according to Claim 1, wherein (C) is a product obtained by fractionating milk serum from a fermentation product, which is produced by lactic acid fermentation of a culture medium mainly containing animal milk by seeding the culture medium with lactic acid bacteria.

7. A cosmetic material according to Claim 1, wherein the lactic acid bacteria are selected from a group consisting of Lactobacillus acidophilus, Lactobacillus bulgaricus, Lactobacillus casei, Streptococcus thermophilus and Lactococcus lactis.

8. A method for preparing a cosmetic material comprising adjusting the pH value of a mixture containing (A) lactic acid, (B) glycolic acid, in a weight ratio of (A) : (B) from 2 : 8 to 9 :1, and (C) a supernatant of a culture medium of lactic acid bacteria to pH 8 to 9; heating the mixture at 80 to 90°C for 1 hour or more; and then adjusting the pH value of the mixture to pH 3.5 to 8.0.

## Patentansprüche

1. Kosmetisches Material, enthaltend (A) Milchsäure, (B) Glycolsäure und (C) einen Überstand eines Kulturmediums von Milchsäurebakterien, wobei das Gewichtsverhältnis von (A):(B) von 2:8 bis 9:1 beträgt.

2. Kosmetisches Material nach Anspruch 1, wobei das Gewichtsverhältnis von (A):(B) von 3:7 bis 8:2 beträgt.

3. Kosmetisches Material nach Anspruch 1, wobei das Gewichtsverhältnis von (A):(B) von 4:6 bis 7:3 beträgt.

4. Kosmetisches Material nach Anspruch 1, wobei (C) in einem Anteil von 3 bis 40 Gew.-% vorhanden ist.

5. Kosmetisches Material nach Anspruch 1, wobei (C) in einem Anteil von 5 bis 20 Gew.-% vorhanden ist.

6. Kosmetisches Material nach Anspruch 1, wobei (C) ein Produkt, erhalten durch Fraktionierung von Milchserum aus einem Gärungsprodukt, ist, welches durch Milchsäuregärung eines Kulturmediums, hauptsächlich enthaltend tierische Milch, durch Impfen des Kulturmediums mit Milchsäurebakterien erzeugt wird.

7. Kosmetisches Material nach Anspruch 1, wobei die Milchsäurebakterien aus einer Gruppe, bestehend aus Lactobacillus acidophilus, Lactobacillus bulgaricus, Lactobacillus casei, Streptococcus thermophilus und Lactococcus lactis, ausgewählt sind.

8. Verfahren zum Herstellen eines kosmetischen Materials, umfassend Einstellen des pH-Werts eines Gemisches, enthaltend (A) Milchsäure, (B) Glycolsäure in einem Gewichtsverhältnis von (A):(B) von 2:8 bis 9:1 und (C) einen Überstand eines Kulturmediums von Milchsäurebakterien, auf pH 8 bis 9; Erwärmen des Gemisches fiir 1 Stunde oder mehr auf 80 bis 90°C und dann Einstellen des pH-Werts des Gemisches auf pH 3,5 bis 8,0.

## Revendications

1. Substance cosmétique contenant de l'acide lactique (A), de l'acide glycolique (B) et un surnageant d'un milieu de culture de bactéries lactiques (C), en quoi la proportion en poids de (A):(B) est de 2:8 à 9:1.

2. Substance cosmétique selon la Revendication 1, en quoi la proportion en poids de (A):(B) est de 3:7 à 8:2.

3. Substance cosmétique selon la Revendication 1, en quoi la proportion en poids de (A):(B) est de 4:6 à 7:3.

4. Substance cosmétique selon la Revendication 1, en quoi (C) est présent en quantité variant de 3 à 40% de poids.

5. Substance cosmétique selon la Revendication 1, en quoi (C) est présent en quantité variant de 5 à 20% de poids.

6. Substance cosmétique selon la Revendication 1, en quoi (C) est un produit obtenu en fractionnant le lacto-sérum à partir d'un produit de fermentation, lequel est produit par fermentation lactique d'un milieu de culture contenant principalement du lait animal, en inoculant le milieu de culture avec des bactéries lactiques.

7. Substance cosmétique selon la Revendication 1, en quoi les bactéries lactiques sont sélectionnées à partir d'un groupe consistant en Lactobacillus acidophilus, Lactobacillus bulgaricus, Lactobacillus casei, Streptococcus thermophilus et Lactococcus lactis.

8. Méthode pour préparer une substance cosmétique comprenant l'ajustement de la valeur de pH d'un mélange contenant de l'acide lactique (A), de l'acide glycolique (B), en proportion en poids de (A): (B) de 2: 8 à 9: 1, et un surnageant (C) d'un milieu de culture de bactéries lactiques, à un pH de 8 à 9; le chauffage du mélange à une température de 80 à 90°C pendant 1 heure ou plus; et ensuite l'ajustement de la valeur de pH du mélange à un pH de 3,5 à 8,0.
